# EUROPÄISCHE PATENTANMELDUNG

(11) **EP 0 835 880 A1**
(43) Veröffentlichungstag der Anmeldung: **15.04.1998**
(21) Anmeldenummer: 96810690.6
(22) Anmeldetag: 14.10.1996
(51) Int. Cl.: C07K 16/06, A61K 39/395

(54) **Verfahren zur Herstellung eines IgM Präparates für die intravenöse Applikation**

(71) Anmelder: ROTKREUZSTIFTUNG ZENTRALLABORATORIUM BLUTSPENDEDIENST SRK, CH-3000 Bern 22 (CH)
(72) Erfinder: Rentsch, Markus, 3400 Burgdorf (CH)
(74) Vertreter: BOVARD AG - Patentanwälte

(57) **Zusammenfassung**

Im Verfahren zur Herstellung einer für die intravenöse Applikation geeigneten Immunglobulinlösung mit einem IgM-Anteil von mehr als 5 Gew.%, bezogen auf den Immunglobulinanteil, wird eine IgM-haltige Immunglobulinlösung mit einer Protease behandelt. Das erhaltene i.v. verträgliche Präparat zeichnet sich dadurch aus, dass es nicht chemisch modifiziert ist und eine niedrige antikomplementäre Aktivität ACA aufweist.

## Beschreibung

Die Erfindung betrifft ein Verfahren zur Herstellung einer für die intravenöse Applikation geeigneten Immunglobulinlösung. Als Ausgangsmaterial wird eine aus humanem oder tierischem Blut erhaltene Proteinfraktion verwendet, die Immunglobuline in ankonzentrierter Form enthält.

Die Immunglobuline spielen bekanntlich im Immunsystem von Mensch und Säugetier bei der Abwehr von Infektionen eine wichtige Rolle. Die Immunglobuline werden in verschiedenen Klassen (z.B. IgG, IgA, IgM, IgD und IgE) mit unterschiedlichen biochemischen und physiologischen Eigenschaften eingeteilt. Bis 1980 wurde lediglich IgG isoliert und als i.v.- verträgliches Produkt für Prophylaxe und Therapie eingesetzt. In EP-A-0 013 901, EP-A-0 413 187 und EP-A-0 352 500 werden IgM Präparate beschrieben, welche hauptsächlich durch Behandlung mit β-Propiolacton intravenös verträglich gemacht wurden. EP-A-0 413 188 beschreibt ein Verfahren, in welchem die i.v. Verträglichkeit durch eine Anionentauscherchromatographie mit selektiver Elution der i.v. verträglichen Fraktion erreicht wird.

Ziel der vorliegenden Erfindung ist die Herstellung eines hochgereinigten, zur intravenösen Verabreichung geeigneten IgM-Konzentrates für die Therapie und Prophylaxe. Das Produkt soll eine niedrige antikomplementäre Aktivität (ACA) und einen tiefen Blutdruckabfall in einem Rattenmodell aufweisen, die IgM-Moleküle sollen aber chemisch nicht modifiziert sein. Dieses Ziel wurde überraschenderweise durch Behandlung einer IgM-haltigen Immunglobulinlösung mit einer Protease erreicht.

Gegenstand der Erfindung ist demzufolge das im Patentanspruch 1 definierte Verfahren.

Vorzugsweise ist die Proteasebehandlung eine bei erhöhter Temperatur durchgeführten Inkubation in Gegenwart von Pepsin, Papain, Plasmin oder Thermolysin. Die Proteasen können auch chemisch modifiziert, an einem Träger immobilisiert und/oder gentechnisch hergestellt sein. Das nach dem erfindungsgemässen Verfahren erhaltene Präparat kann in eine i.v. verabreichbare Lösung übergeführt werden. Eine solche zeigt eine Verminderung der ACA, des Blutdrucksabfalls in einem Rattenmodell und der C1q-Bindungsaktivität.

Als Ausgangsmaterialien für das Verfahren gemäss der vorliegenden Erfindung eignen sich immunglobulinhaltige Lösungen, wie z.B. Plasma, Niederschlag A oder B aus Kistler-Nitschmann-Fraktionierung; Cohn Fraktion I/II/III; II/III, III; oder andere IgM-haltige Plasmafraktionen aus humanem oder tierischem Plasma. Zum Beispiel kann man eine immunglobulinhaltige Fraktion, wie Niederschlag B nach Kistler-Nitschmann, in Puffer lösen, die meisten Verunreinigungen durch eine Fällung mit 0,5 bis 5 % Octansäure bei pH 4 bis 6, vorzugsweise pH 5 entfernen. Anschliessend wird die Lösung bei tiefer Ionenstärke 1 bis 48 Stunden, vorzugsweise 9 Stunden bei einer Temperatur von 20 bis 50 °C, vorzugsweise 37 °C unter Zusatz von mindestens 50 U/g Pepsin, vorzugsweise 600 U/g inkubiert.

Zur weiteren Reinigung kann die Lösung einer Adsorption unterworfen werden, beispielsweise mit einem DEAE-Gruppen enthaltendem Gel im Batch oder Säulenverfahren. Soll die IgM-Konzentration im Endprodukt weiter erhöht werden, so wird die IgM-haltige Lösung auf einen Ionentauscher (z.B. TMAE-Fraktogel®) aufgezogen. Durch eine selektive Elution z.B. mittels eines Salz- oder pH-Gradienten kann die IgM-Fraktion isoliert werden. Durch Ultra- und Diafiltration, beziehungsweise eine Gelfiltration, kann die Lösung konzentriert und der Elektrolytgehalt auf die endgültige intravenös verträgliche Formulierung eingestellt werden. Die Proteinkonzentration kann 1 bis 20 %, vorzugsweise 3 bis 6 % betragen. Das Produkt kann zusätzlich Proteine, vorzugsweise Albumin, sowie Zucker, vorzugsweise Glucose oder Saccharose, oder Aminosäuren enthalten.

Zur Beurteilung der intravenösen Verträglichkeit von Immunglobulinpräparaten wird üblicherweise die antikomplementäre Aktivität (ACA) herangezogen. Zur Bestimmung der ACA wird eine definierte Menge des zu prüfenden Produktes mit einer definierten Menge von Meerschweinchen-Komplement inkubiert und die verbleibende Menge Komplement titriert. Die ACA wird als Verbrauch der CH50 pro g Immunglobulin angegeben. Die angegebenen Resultate der ACA wurden weitgehend nach der von M. Mayer publizierten Methode bestimmt. (Mayer, M.M. (1961) Complement and complement fixation. In: Experimental Immunochemistry, 2nd edn., pp 133-240, C Thomas, Springfield, II). Als Richtwert für intravenös anwendbare IgG-Produkte gilt eine ACA von < 1000 CH50 pro g Protein.

Zur Beurteilung der intravenösen Verträglichkeit kann weiter die Bindung der C1q-Komplementkomponente an das Immunglobulin herangezogen werden. Zur Bestimmung wird eine definierte Menge Prüfprodukt mit einer definierten Menge gereinigtem, radioaktiv markiertem C1q-Komplement in Puffer und in Serum inkubiert. Die C1q-Bindungsaktivität des Prüfproduktes wird durch Präzipitation in Gegenwart von Polyäthylenglykol ermittelt. Je höher die Radioaktivität im Niederschlag ist, umso grösser ist die C1q-Bindungsaktivität des Produktes. Verfeinerte Aussagen über die Art der C1q-Bindung und damit die Qualität des Produktes lassen sich erzielen, wenn das C1q mit zwei verschiedenen Methoden radioaktiv markiert wird. Einerseits unter möglichst milden oxidativen Bedingungen mit Lactoperoxidase (LPO) und andererseits unter drastischen oxidativen Bedingungen mit Chloramin T (CT). Die Untersuchungen wurden weitgehend nach der von P.Späth publizierten Methode durchgeführt. (P.J.Späth, A.Corvetta, U.E.Nydegger, R.Büttler: An Extended C1q-Binding Assay Using Lactoperoxidase- and Chloramin-T-Iodinated C1q. Scand.J.Immunol. 18, 319-328, 1983) Von einem intakten, intravenös verträglichen Präparat wird erwartet, dass die C1q-Bindungsaktivität möglichst gering ist. Ein Modell zur Prüfung der i.v. Verträglichkeit von Immunglobulinen ist das Rattenmodell nach Bleeker et al. [W.K.Bleeker, J. Agterberg, G.Rigter, A. de Vries-van Rossen, J.C.Bakker: An animal model for the detection of hypotensive side effects of immunoglobulin preparations. Vox.Sang. 52: 281 - 290 (1987)]. Verträglichkeitsparameter in diesem Modell ist der Blutdruck. Intravenös unverträgliche Produkte führen zu einer deutlichen Blutdrucksenkung.

### Beispiele

### Referenzbeispiel 1

1 kg Niederschlag B nach Kistler-Nitschmann wurde in 4 kg 0,1 M Acetatpuffer, pH 5,1 suspendiert und mit 2 % Octansäure bei Raumtemperatur versetzt. Pro g Octansäure wurden 0,15 g Tricalciumphosphat zugegeben und der Niederschlag abfiltriert. Das Filtrat wurde gegen 20 mmol/l Piperazin, 60 mmol/l NaCl, pH 5,8 diafiltriert. Die diafiltrierte Lösung wurde mit 75 mg DEAE-Sephadex® pro g Protein behandelt. Anschliessend wurde die Proteinkonzentration auf 20 mg/ml eingestellt und die Lösung mit 1 % Tween® 80 und 0,3 % TNBP (Tri-n-butyl-phosphat) 8 Stunden bei 25 °C behandelt. Die Lösung wurde anschliessend auf eine TMAE-Fraktogel®-Säule aufgezogen und die IgM-Fraktion mit 20 mmol Piperazin, 160 mmol NaCl, pH 5,8 eluiert. Das Endprodukt wurde auf 5 % Protein aufkonzentriert und der pH-Wert auf 4,5 eingestellt.

### Referenzbeispiel 2

1 kg Niederschlag B nach Kistler-Nitschmann wurde in 4 kg 0,1 M Acetatpuffer, pH 5,1 suspendiert und mit 2 % Octansäure bei Raumtemperatur versetzt. Pro g Octansäure wurden 0,15 g Tricalciumphosphat zugegeben und der Niederschlag abfiltriert. Das Filtrat wurde gegen 20 mmol/l NaCl diafiltriert und die Lösung auf 20 mg/ml Protein gebracht. Der pH-Wert wurde mit 0,2 M HCl auf 4,0 eingestellt und die Lösung während 9 Stunden bei 37 °C inkubiert. Nach Abkühlen auf 20 °C wurde das pH auf 5,8 eingestellt und Piperazin ad 20 mmol/l und NaCl ad 60 mmol/l zugegeben. Die Lösung wurde danach mit 1 % Tween® 80 und 0,3 % TNBP (Tri-n-butyl-phosphat) 8 Stunden bei 25 °C behandelt. Die Lösung wurde anschliessend auf eine TMAE-Fraktogel®-Säule aufgezogen und die IgM-Fraktion mit 20 mmol Piperazin, 160 mmol NaCl, pH 5,8 eluiert. Das Endprodukt wurde auf 5 % Protein aufkonzentriert und der pH-Wert auf 4,5 eingestellt.

### Beispiel 1

1 kg Niederschlag B nach Kistler-Nitschmann wurde in 4 kg 0,1 M Acetatpuffer, pH 5,1 suspendiert und mit 2 % Octansäure bei Raumtemperatur versetzt. Pro g Octansäure wurden 0,15 g Tricalciumphosphat zugegeben und der Niederschlag abfiltriert. Das Filtrat wurde gegen 20 mmol/l NaCl diafiltriert und die Lösung auf 20 mg/ml Protein gebracht. Der pH-Wert wurde mit 0,2 M HCl auf 4,0 eingestellt und es wurden 600 U Pepsin pro g Protein zugegeben. Dann wurde die Lösung während 9 Stunden bei 37 °C inkubiert. Nach Abkühlen auf 20 °C wurde das pH auf 5,8 eingestellt und Piperazin ad 20 mmol/l und NaCl ad 60 mmol/l zugegeben. Die Lösung wurde danach mit 1 % Tween® 80 und 0,3 % TNBP (Tri-n-butyl-phosphat) 8 Stunden bei 25 °C behandelt. Die Lösung wurde anschliessend auf eine TMAE-Fraktogel®-Säule aufgezogen und die IgM-Fraktion mit 20 mmol Piperazin, 160 mmol NaCl, pH 5,8 eluiert. Das Endprodukt wurde auf 5 % Protein aufkonzentriert und der pH-Wert auf 4,5 eingestellt.

### Beispiel 2

1 kg Niederschlag B wurde analog Refenzbeispiel 2 aufgearbeitet, wobei jedoch anstelle von 600 U Pepsin pro g Protein der Lösung 1200 U Pepsin pro g Protein vor der pH 4-Inkubation zugegeben wurden.

### I. Charakterisierung der experimentellen Produkte

Die Immunglobuline IgG, IgA und IgM wurden nephelometrisch mit Antiseren bestimmt. Der Totalproteingehalt wurde mit der Kjeldahl-Methode bestimmt.

| | Protein mg/g | IgG mg/g | IgA mg/g | IgM mg/g | Isoagglutinine | |
|---|---|---|---|---|---|---|
| | | | | | Anti-A | Anti-B |
| Referenzbeispiel 1 | 44,4 | 4,1 | 16,8 | 45,0 | 1:64 | 1:64 |
| Referenzbeispiel 2 | 48,1 | 8,6 | 19,3 | 43,5 | 1:128 | 1:64 |
| Beispiel 1 | 51,3 | 7,0 | 22,0 | 50,5 | 1:128 | 1:64 |
| Beispiel 2 | 46,9 | 4,7 | 20,2 | 51,0 | 1:128 | 1:64 |

### II. Tabelle Verträglichkeitsparameter

| | Behandlung | ACA | Rattenmodell | C1q-Bindung | | | |
|---|---|---|---|---|---|---|---|
| | | | | Puffer | | Serum | |
| | | CH50/g | Blutdruckabfall % | LPO % | CT % | LPO % | CT % |
| Referenzbeispiel 1 | ohne pH 4 | 515 | 19 | 1,5 | 0,1 | 43 | 5,5 |
| Referenzbeispiel 2 | pH 4 | 179 | 18 | 0 | 0,5 | 42 | 6,7 |
| Beispiel 1 | pH 4 mit 600 U Pepsin | 125 | 7 | 0 | 0,3 | 34 | 3,9 |
| Beispiel 2 | pH 4 mit 1200 U Pepsin | 89 | 2 | 0 | 0,5 | 23 | 3,7 |

Der Zusatz von Pepsin bewirkt eine Reduktion der ACA, eine Verminderung des Blutdruckabfalles im Rattenmodell, sowie eine Senkung der C1q- Bindungsaktivität.

## Patentansprüche

1. Verfahren zur Herstellung eines intravenös verabreichbaren polyklonalen, chemisch nicht modifizierten Immunglobulinpräparates mit einem IgM-Anteil von mehr als 5 Gew.%, bezogen auf den Gesamtimmunglobulinanteil, und einer niedrigen antikomplementären Aktivität, dadurch gekennzeichnet, dass eine wässerige Lösung oder Suspension einer entsprechenden IgM-haltigen Plasmafraktion mit einer Protease behandelt wird.

2. Verfahren nach Anspruch 1, dadurch gekennzeichnet, dass das Präparat eine antikomplementäre Aktivität von < 500 CH50 /g Protein, bevorzugt von < 200 CH50 /g Protein und insbesondere von < 150 CH50 /g Protein aufweist.

3. Verfahren nach Anspruch 1 oder 2, dadurch gekennzeichnet, dass die wässerige Lösung oder Suspension der IgM-haltigen Plasmafraktion bei einem sauren pH-Wert unter Zusatz einer Protease bei einer Temperatur von mindestens 15 °C inkubiert wird.

4. Verfahren nach Anspruch 3, dadurch gekennzeichnet, dass die Inkubationstemperatur 20 bis 50 °C, vorzugsweise 35 bis 40 °C beträgt.

5. Verfahren nach Anspruch 3 oder 4, dadurch gekennzeichnet, dass die Inkubationsdauer 1 bis 48 Stunden, vorzugsweise 6 bis 12 Stunden beträgt.

6. Verfahren nach einem der Ansprüche 1 bis 5, dadurch gekennzeichnet, dass die Proteasekonzentration in der wässerigen Lösung oder Suspension der IgM-haltigen Plasmafraktion mindestens 50 U/g Protein, vorzugsweise 300 bis 1200 U/g beträgt.

7. Verfahren nach einem der Ansprüche 1 bis 6, dadurch gekennzeichnet, dass der pH-Wert der wässerigen Lösung oder Suspension der IgM-haltigen Plasmafraktion bei der Proteasebehandlung 3,5 bis 5,5, vorzugsweise 3,7 bis 4,3 beträgt.

8. Verfahren nach einem der Ansprüche 1 bis 7, dadurch gekennzeichnet, dass die Protease eine Endopepdidase ist.

9. Verfahren nach Anspruch 8, dadurch gekennzeichnet, dass die Protease mindestens eine Endopeptidase ist, die ausgewählt ist aus der Gruppe Pepsin, Papain, Plasmin oder Thermolysin, die gegebenenfalls an einem Träger immobilisiert sein können.

10. Verfahren nach einem der Ansprüche 1 bis 9, dadurch gekennzeichnet, dass die Ionenstärke in der wässerigen Lösung oder Suspension der IgM-haltigen Plasmafraktion < 0,1, vorzugsweise < 0,04 ist.
